Europäisches Patentamt

European Patent Office

Office européen des brevets

(11) Publication number: **0 661 044 A1**

# EUROPEAN PATENT APPLICATION

(21) Application number: **95102781.2**

(22) Date of filing: **03.07.91**

(51) Int. Cl.⁶: **A61K 9/127**, A61K 31/475

This application was filed on 27 - 02 - 1995 as a divisional application to the application mentioned under INID code 60.

(43) Date of publication of application:
**05.07.95 Bulletin 95/27**

(60) Publication number of the earlier application in accordance with Art.76 EPC: **0 592 446**

(84) Designated Contracting States:
**AT BE CH DE DK ES FR GB GR IT LI LU NL SE**

(71) Applicant: **VESTAR, INC.**
**650 Cliffside Drive**
**San Dimas**
**California 91773 (US)**

(72) Inventor: **The designation of the inventor has not yet been filed**

(74) Representative: **Brown, David Leslie et al**
**Page Hargrave**
**Temple Gate House**
**Temple Gate**
**Bristol BS1 6PL (GB)**

(54) **Drug-containing liposomes.**

(57) Unilamellar liposomes having a diameter of from 30 to 150 nm are described, comprising a liposomal membrane including distearoylphosphatidylcholine (DSPC) and cholesterol, in a DSPC:cholesterol molar ratio of about 2:1, the liposomes encapsulating an antineoplastic selected from vincristine and vinblastine and an anion of low liposomal membrane permeability including a carboxylate moiety, for example a monocarboxylate, dicarboxylate, tricarboxylate or polycarboxylate anion, more particularly gluceptate, fumarate, gluconate, lactobionate, maleate, succinate, glutarate, tartrate, citrate or carbopol.

EP 0 661 044 A1

Rank Xerox (UK) Business Services
(3.10/3.09/3.3.4)

## Field of the Invention

This invention relates to the field of biochemistry and medicine, and more particularly to a method of preparing pharmaceutical preparations which include liposome-entrapped lipophilic drug formulations, and to methods of using such formulations as therapeutic agents to control the delivery of encapsulated drugs within the bodies of mammals, including humans.

## Background of the Invention

Liposomes are known to be physiologically compatible and biodegradable delivery systems for a broad range of drugs. For example, U.S. Patent 5,019,369 teaches the ability to target diagnostic and chemotherapeutic agents to tumors in the body of a patient by the intravenous administration of the agent encapsulated in unilamellar liposomes having a diameter of less than 200 nm.

Liposomes are microscopic delivery vesicles that are comprised of amphiphilic molecules such as phospholipids, which are polar molecules having a hydrophilic (ionizable) headgroup and a hydrophobic tail consisting of fatty acid chains. Phospholipids form closed, fluid filled spheres when properly mixed with water. The hydrophobic tails spontaneously associate and exclude the water, while the hydrophilic phosphate ester headgroups are preferentially positioned toward the water.

The result is a spherical bilayer membrane in which the fatty acid tails point towards the interior of the membrane, and the polar heads are arranged on the membrane surface, pointing toward an aqueous medium. For single bilayer membranes, the polar heads at the inner surface of the membrane point toward the liposome's aqueous interior and those at the other (outer) surface point toward the exterior aqueous medium (*i.e.*, the external continuous phase of the liposome dispersion). Liposomes may be either multilamellar, like an onion, with water separating the many lipid bilayers, or unilamellar, with a single bilayer surrounding an aqueous center.

It is the ability of phospholipids to form liquid filled spheres that provided the first method for loading liposomes with medically active agents. As the liposomes form, water soluble molecules dissolved in the solution in which the phospholipids are dispersed will be incorporated into the aqueous interior and thus be passively encapsulated within the liposomes.

One drawback associated with these methods is the low level of drug encapsulation (trapping efficiency) achievable by such methods. PCT patent publications WO86/01102 and WO86/01103 disclose active loading methods which permit trapping efficiencies approaching 100% encapsulation while, at the same time, increasing the rate at which the drug is loaded into the liposome carrier. These methods involve generating transmembrane potentials to create a gradient for one or more charged species (*e.g.,* $Na^+/K^+$, $Ca^{++}$ and $H^+$ are disclosed) across the walls of the liposome. The concentration gradient, as the name implies, results from producing liposomes having different concentrations of charged species within (the internal phase) and without (the external phase) the vesicles. A disadvantage of this technique is that it requires the inclusion of an ionophore or other such transporter molecule within the vesicle membrane in order to move the charged ions across the lipid bilayer, thus allowing for exchange of the ions for the drug to be encapsulated (loaded). The inclusion of certain ion transporting agents within the lipid membrane can alter the *in vivo* biodistribution of the vesicle carrier, potentially increasing undesirable uptake by the spleen, liver and/or other organs of the reticuloendothelial system or otherwise interfering with uptake into desired tissue sites. An additional and potentially undesirable effect of this approach is that in order to obtain efficient drug encapsulation, a high ionic gradient must be created across the lipid bilayer which may stress and destabilize the liposome membrane.

An alternate approach to the use of ionic gradients has been the application of pH ($H^+$) gradients to load drugs or other agents of interest into preformed liposomes. This technique has been described in the literature, for example, U.S. Pat. No. 4,946,683; and European Patent Office publication No. 0290296. A particular disadvantage of using pH gradients for loading drugs into liposomes, especially in large scale-manufacturing, is that significant lipid hydrolysis can occur as a result of exposing the lipids to extreme pH changes during the loading process. Unstable lipid domains may also form within membrane bilayers resulting from changes in the electrical charge and/or hydration states of the lipid head groups due to protonation-deprotonation of titratable moieties in the lipids. In order to most efficiently load and take advantage of the maximum ionization potential of a counterion, pH changes on the order of 1.5-2 units or more are typically required. This could preclude use of some acidic drugs with $pK_a$s less than about 5.0 or basic drugs with $pK_b$s greater than about 8.0 as counterions for drug loading. For example, if one wished to encapsulate a cationic (+) compound such as an amine into vesicles containing an anionic (-) counter ion, phosphate or citrate could not be used efficiently. 0.1 M solutions of phosphoric ($H_3PO_4$; $pK_{a1} = 2.15$, $pK_{a2} = 7.10$, $pK_{a3} = 12.32$) or citric ($C_6H_8O_7$; $pK_{a1} = 3.13$, $pK_{a2} = 4.76$, $pK_{a3} = 6.40$)

acids would have pHs of about 1.5 and 2.2, respectively. Preparation of lipid vesicles in solutions at these pHs would result in excessive and unacceptable lipid hydrolysis. As a second example, if it were desirable to encapsulate an anionic (-) compound such as an amine into vesicles containing a cationic ( + ) counter ion, strong bases such as sodium or calcium hydroxides likewise would not be desirable due to excessive lipid hydrolysis resulting from high pHs. In addition, calcium hydroxide is also of limited aqueous solubility.

One proposed approach for avoiding pH extremes imposed by pH loading methods has been noted in European Patent Office publication 0361894. In this method, pH gradients are generated by differences in transmembrane ammonium ($NH_4$) ion concentrations. An advantage of this technique is that the loading process can be conducted at or near neutral pHs without the necessity of exposing the preparation to severe pH ranges. Neutral ammonia exits the internal aqueous space through the membrane bilayer to create a pH gradient. This is followed by the influx of a deprotonated amphiphilic drug to replace the departed ammonium. One disadvantage of this method is that during large scale-manufacturing it can be difficult to control ammonium concentrations at each step of the manufacturing process. This is in part due to the volatility of ammonia which can readily evaporate during high temperature processing thus allowing ammonium salts to revert back to their corresponding acid forms.

Accordingly, it has been a desideratum to provide an active loading method for the encapsulation of agents within liposomes which avoids the use of adverse pH ranges and gradients and will be readily adaptable to large scale-manufacturing techniques.

## Summary of the Invention

The invention is an ion-exchange loading technique for incorporating a cationic or anionic lipophilic drug, which can partition into or through a lipid bilayer, into lipid vesicles, *e.g.*, unilamellar or multilamellar liposomes. This technique promotes the passage of the drug through the lipid membrane into the internal aqueous space within the liposome, thereby increasing entrapment efficiency and thus the amount of the drug which can be administered and delivered within the body, *e.g.*, the amount of an antineoplastic agent delivered specifically to tumor tissues. In particular, the method provides an enhanced ability to produce these drug delivery formulations on a large scale.

According to the practice of the invention, membrane permeable drugs or other compounds of interest are preferably loaded into preformed liposomes in sufficiently high quantities to be useful for therapeutic applications. In another aspect of the invention, the ion-exchange loading process is performed in the absence of a pH gradient, which provides significant advantages in the large scale production of lipid vesicles. Preferably, the loading occurs in the absence of an ionophore within the liposome membrane.

The process comprises the formation of liposomes in an aqueous medium containing a combination of either the salt of an anion with high membrane permeability and a cation with low membrane permeability, if the compound to be entrapped is an anionic drug or has an anionic potential; or the salt of a cation with high membrane permeability and an anion with low membrane permeability, if the compound to be entrapped is a cationic drug or has a cationic potential.

As used herein, unless limited by qualifying language, the terms cation and anion include those ions having cationic or anionic charges in water at neutral pH, and also includes neutral moieties which become cationic or anionic under the loading reaction conditions. For example, the drug doxorubicin is an essentially neutral molecule which becomes cationic at acid to neutral pHs.

In one aspect of the invention, a method is provided for loading a cationic substance into liposomes. The method comprises forming liposomes in an aqueous medium including a metal-free exchange cation of high liposomal membrane permeability containing carbon and nitrogen, and an anion of low liposomal membrane permeability, to produce liposomes which include a portion of the medium containing the cation and anion in the interior aqueous space and which are dispersed in the aqueous medium which is external to the liposomes. Preferably, the exchange cation is an amine, and most preferably a primary, secondary or tertiary amine. The concentration of the cation and anion in the external aqueous medium is then lowered, such as by ultrafiltration or other buffer exchange means, and the cationic substance to be loaded is added to the external phase of the dispersion.

In another aspect of the invention, a method is provided for loading an anionic substance into liposomes. The method comprises forming liposomes, in an aqueous medium having a given pH and containing an exchange anion of high liposomal membrane permeability and a cation of low liposomal membrane permeability, to produce liposomes which include a portion of the medium containing the anion and cation in the interior aqueous space and which are dispersed in the aqueous medium which is external to the liposomes. The concentration of the anion and cation in the external aqueous medium is lowered while maintaining the pH of the medium equal to that of the interior aqueous space, and the anionic substance to be loaded is added to the external phase of the dispersion.

An additional substance, such as a chelator or chelatable material that is capable of binding to the exchange ion with high membrane permeability noted above while not binding to the compound of interest, can also be included in or added to the external aqueous phase in order to trap the permeable ion thus assisting in driving equilibrium towards complete (100%) entrapment.

## Detailed Description of the Invention

Any cationic, lipophilic or amphiphilic drug which can partition into a lipid bilayer is suitable for use in practicing this invention, *e.g.*, dibucaine, pilocarpine, quinine, prodipine, timolol, pentamidine, benadryl, dopamine, epinephrine, codeine, morphine, atropine, imipramine, quinidine, chloropromazine, and others.

Cationic compounds having antineoplastic activity against cancerous tissues or cells, including daunorubicin, doxorubicin, aclacinomycin A, vinblastine, vincristine, mitomycin C, mitoxantrone, and the like, are particularly preferred for incorporation within liposome micellar particles using the procedure of this invention.

Anionic drugs which can pass through into a lipid membrane, *e.g.*, penicillins, ampicillin, chlorambucil, warfarin, cephalothin, phenobarbital, and succinate hemiesters are suitable for use in the method of the invention.

Biological lipids from which liposome bilayer membrane particles or vesicles useful in practicing this invention can be prepared are amphiphilic (containing both a lipophilic and hydrophilic portion) molecules which can spontaneously aggregate to form small spheres, ellipsoids or long cylinders, or bilayers having two or more parallel layers of amphiphilic molecules. In an aqueous (polar) medium, the polar heads of the amphiphilic molecules making up one layer orient outwardly to extend into the surrounding medium while the non-polar tail portions of these molecules likewise associate with each other. This provides a polar surface and a non-polar core in the wall of the vesicle. Such bilayered micelles usually take the shape of unilamellar (having one bilayer) or multilamellar (having a plurality of substantially concentric bilayers) spherical vesicles having an internal aqueous compartment.

Liposome bilayer membrane particles which have been found to be suitable in practicing this invention are small unilamellar vesicles having a diameter of from 30 to 150 nanometers (nm), and preferably from about 45 to about 60 nm, which are neutral (uncharged or having balanced charges; *i.e.*, zwitterions) to induce specificity and tissue/cell targeting, thereby maximizing therapeutic uptake of the liposome drug delivery system.

Such liposome bilayer membrane particles include ones made from dipalmitoyl phosphatidylcholine, distearoyl phosphatidylcholine, dioleoyl phosphatidylethanolamine, distearoyl phosphatidylserine, dilinoleoyl phosphatidylinositol, distearoyl phosphatidylglycerol, and the like, or mixtures thereof. Liposome bilayer membrane particles made entirely from neutral phospholipids, such as distearoyl phosphatidylcholine, and preferably ones which have been further stabilized with cholesterol or like-acting substances, for example in a molar ratio of distearoyl:phosphatidylcholine:cholesterol of about 2:1, respectively, have been found to be particularly suitable with regard to targeting efficiency when used to deliver anthracycline antineoplastic agents.

These liposomes are prepared by generally known techniques, such as the sonication method described in Mauk et al, *Anal. Bioc.* 94. 302-307 (1979) or preferably by microemulsification using the procedure described in U.S. Patent No. 4,753,788, the disclosures of which are incorporated by reference herein. Homogenization using a sonicator device will generally be carried out for from about 30 seconds to one minute per milliliter of suspension. Following homogenization, the suspension is centrifuged at from about 1,000 xg to about 20,000 xg, and preferably at about 5,000 xg, for from about 5 to 20 minutes, preferably about 10 minutes, at ambient temperature (usually about 22°C.), and then passed through a small pore diameter sterile filter, *e.g.*, a 0.2-0.45 micron pore filter. These two steps (centrifugation and filtration) remove large particulate matter such as unsuspended lipids, large vesicles and other possibly contaminating particles.

As set forth above, the invention comprises a method for loading an anionic or cationic substance into liposomes. First, liposomes are formed in an aqueous medium containing either an anion with high membrane permeability and a cation of low membrane permeability if the substance to be entrapped is an anion or can be converted to an anion; or a cation with high membrane permeability and an anion of low membrane permeability if the substance to be entrapped is a cation or can be converted to a cation. This produces a dispersion of liposomes in the aqueous medium which include the respective anion and cation in the interior space of the liposomes. Then, the concentration of the anion and cation in the aqueous medium is lowered, and the substance to be loaded into the internal aqueous space of the liposomes is added to the external phase of the liposome dispersion. In summary, the method comprises forming liposomes having a higher concentration of the salt of an anion with high membrane permeability and a cation of low membrane permeability if the substance to be entrapped is an anion, or the salt of a cation with high membrane permeability and an anion of low membrane permeability if the substance to be entrapped is a cation in the internal aqueous space and in the external aqueous phase of the liposomes; and adding the anionic or cationic substance to the liposome dispersion to load the substance into the internal aqueous space of the liposomes.

In situations in which the substance to be entrapped is a cation, the cation which is present in the internal aqueous phase in high concentration preferably contains one or more nitrogen atoms, and most preferably is a primary, secondary or tertiary amine. Specifically, the cation which effects the loading of the cationic substance is a methylamine (including methylamine, dimethylamine and trimethylamine), an ethylamine (including ethylamine, diethylamine and triethylamine), a propylamine, ethylenediamine or ethanolamine. In this aspect of the loading, the anion is preferably electrically charged throughout the range of 3 through 11 pH units; preferably is a compound which includes sulfate, sulfonate, phosphate, phosphonate and/or carboxylate moieties. Specifically, the anion is a mono-, di-, tri-, or polycarboxylate; gluceptate, gluconate, lactobionate, maleate, succinate, glutarate, tartrate, citrate or carbopol; or the anion may be a phosphate, sulfate, mesylate, estylate; such as dextran sulfate or heparin sulfate.

In instances in which the substance to be loaded is an anion or can be converted to an anion, the anion which is present in the internal aqueous space in a higher concentration than that in the external aqueous phase is a halide, carbonate or organic acid anion and most preferably a chloride, bromide, fluoride, carbonate, acetate, formate or propionate. The corresponding cation is an alkali metal, alkaline earth, or quaternary ammonium compound. Most preferably, the cation in this instance is sodium, potassium, magnesium, calcium or choline; or is a cation which is electrically charged throughout the range of 2 to 12 pH units.

For the loading of either a cationic or anionic substance, an acid or basic substance can be added to the liposome dispersion during the loading step in amounts which are sufficient to maintain the pH of the continuous aqueous phase at a value equal to that of the internal aqueous space, if desired.

Permeability or leakage can be measured by separating the vesicles from any material which has leaked out, using methods such as gel permeation chromatography, dialysis, ultrafiltration or the like, and assaying in known manner for any leaked material. Permeabilities ranging from about one to about ten percent of the original entrapped material over a period of about 24 hours or longer, and preferably less than about one percent of the original entrapped material, are acceptable.

The following information exemplifies the process of the the invention.

**Example 1**

This example details the entrapment of daunorubicin in distearoyl phosphatidylcholine-cholesterol vesicles using ethylenediamine citrate $(C_2H_8N_2)_3$ $(C_6H_8O_7)_2$.

A dry powder containing a homogenous dispersion of cholesterol and distearoylphosphatidylcholine at a 1:2 molar ratio was prepared by evaporation from a 1:1 chloroform/methanol solution. This dispersion was hydrated with an aqueous solution containing 125 mM lactose and a 25 mM salt of ethylenediamine citrate (75 mM ethylenediamine and 50 mM citrate). The lipid concentration in the resulting suspension was about 20 to 25 mg/ml. This mixture was then heated to 65°C. Next, while maintaining that temperature, the mixture was homogenized using a sonicator to produce a suspension of small unilamellar vesicles. The vesicles thus obtained had diameters principally in the range of about 40 to 80 nm with little or no particles in excess of 100 nm.

Following homogenization, the mixture underwent a buffer exchange to remove the external, unentrapped, ethylenediamine citrate. This was accomplished by subjecting the mixture to tangential flow ultrafiltration. This process removes water and small molecular weight solutes while retaining lipid vesicles and their contents. Using this process, the lactose/ethylenediamine citrate buffer external to the vesicles was replaced with 250 mM lactose/50 mM glycine. The vesicle preparation was heated to about 65°C and an amount of daunorubicin (base or suitable salt form) was added to yield a lipid to drug ratio in the range of about 16:1 to 19:1 or more (w/w), but preferably 17.4:1. If daunorubicin had been added in its hydrochloride salt form and maintenance of the pH of the continuous phase at the original value is desired, it would be necessary to add sufficient base (such as NaOH, NaHCO$_3$ or Na$_2$CO$_3$) to maintain the pH at the above stated value, approximately 6.2. The drug-vesicle mixture was maintained at 65°C for about 10 minutes. During this phase of the procedure, daunorubicin passes through the membrane bilayer of the liposome to form a salt and/or to complex with entrapped citrate. At the same time, ethylenediamine dissipates to the vesicles' exterior aqueous space. Typically, if an appropriate lipid:drug ratio has been selected (approximately at least 16:1, mol/mol), greater than about 90% of the added daunorubicin will become vesicle entrapped as daunorubicin-citrate. If necessary, unentrapped, residual daunorubicin can be removed by methods including ion-exchange, ultrafiltration, gel permeation chromatography or other suitable means used to separate small molecular weight subatances or charged species from compounds with larger molecular weights. Following drug entrapment, the pH of the external phase may be adjusted as required to produce or maintain optimal chemical stability and/or to minimize vesicle aggregation. The formation of a pH gradient between the internal aqueous space and the external continuous phase is not necessary, however, in order to bring about drug loading.

The daunorubicin-vesicle suspension was sterilized by passage through a 0.22 $\mu$m diameter pore filter. The finished vesicle suspension was then ready for use.

As a further example, the procedure of Example 1 may be repeated except that the ethylenediamine citrate is replaced with 75 mM ethylenediamine tartrate. A lipid vesicle suspension containing entrapped daunorubicin-tartrate is obtained.

Further, the procedure of Example 1 was repeated except that following the buffer exchange procedure and drug addition, the chelating/chelatable complex calcium carbonate ($CaCO_3$) was added to the production mixture. $Ca^{++}$ can be chelated by citrate or tartrate; while $CO_3^=$ has a high affinity for ethylenediamine. A lipid vesicle suspension containing entrapped daunorubicin-citrate (or daunorubicin-tartrate as applicable) was thus obtained.

## Example 2

This example describes the entrapment of doxorubicin into distearoyl: phosphatidylcholine:cholesterol vesicles using ethylamine lactobionate.

A dry powder containing a homogenous dispersion of cholesterol in distearoyl:phosphatidylcholine at a 1:2 molar ratio was prepared. This dispersion was hydrated with an aqueous solution containing 50 mM sucrose, 150 mM ethylamine lactobionate, and about 20-30 mM ethylamine, added to adjust pH to 6.2. The lipid concentration in the resulting suspension was about 20 to 25 mg/ml. This mixture was heated to 65°C and homogenized as described in Example 1 and cooled to room temperature.

Next, unentrapped ethylamine lactobionate was removed by buffer exchange as described in Example 1 and replaced with 300 mM sucrose. The vesicle preparation was heated to 65°C and an amount of doxorubicin was added to yield a lipid to drug ratio of 25:1 (w/w), although a range of about 20:1 to 30:1 or more (w/w) is acceptable. Since doxorubicin was added in its hydrochloride salt form it was necessary to add sufficient $Na_2CO_3$ to maintain a constant pH, approximately 6.2. The drug-vesicle mixture was maintained at 65°C for about 10 minutes. The remainder of the processing steps were similar to those described in Example 1. A suspension of unilamellar vesicles having diameters of less than 100 nm containing entrapped doxorubicin-lactobionate was thus obtained.

## Example 3

As another example of the invention, chlorambucil is encapsulated into distearoyl:phosphatidylcholine:cholesterol vesicles using N-methyl-glucamine hydrochloride.

A dry powder containing a homogenous dispersion of cholesterol in distearoyl phosphatidylcholine at a 1:2 molar ratio is prepared. This powder is hydrated with an aqueous solution containing 150 mM N-methyl-glucamine hydrochloride. The lipid concentration in the resulting suspension is about 20 to 25 mg/ml. This mixture is then heated to 65°C. Next, while maintaining that temperature, the mixture is vigorously homogenized using a sonicator to produce a suspension of small unilamellar vesicles. The vesicles so obtained have diameters principally in the range of about 40 to 80 nm with little to no material in excess of 100 nm.

Following homogenization, the mixture undergoes a buffer exchange by tangential flow ultrafiltration to remove the external, unentrapped, N-methyl-glucamine hydrochloride. With this process, the N-methyl-glucamine hydrochloride buffer external to the vesicles is replaced with a low ionic strength solution such as 250 mM lactose/50 mM glycine or 300 mM sucrose. The vesicle preparation is again heated to about 65°C and an amount of chlorambucil is added to yield a lipid to drug ratio in the range of about 16:1 to 19:1 or more (w/w), but preferably 17.4:1. If chlorambucil is added in the free acid form it may be necessary to add sufficient base such as NaOH, $NaHCO_3$ or $Na_2CO_3$ to maintain the pH of the external phase at the value of that of the internal aqueous space. The drug-vesicle mixture should be maintained at 65°C for about 10 minutes to facilitate loading. During this phase of the procedure, chlorambucil will pass through the membrane bilayer to form a salt or complex with entrapped N-methyl-glucamine while the more volatile hydrochloride dissipates to the vesicles' exterior aqueous space. If a sufficient lipid:drug ratio has been selected (approximately at least 16:1 or above), greater than about 90% of the added chlorambucil will become vesicle entrapped. Unentrapped, residual, chlorambucil can be removed by ion-exchange, ultrafiltration, gel permeation chromatography or other suitable means used to separate small molecular weight or charges solutes from substances with larger molecular weights. Following drug entrapment, pH may be adjusted to below 6.0 or above 7.5 as needed to maintain optimal chemical stability or to minimize vesicle aggregation

The chlorambucil-vesicle suspension can then be sterilized by passage through 0.45 $\mu$m or, preferably, 0.22 $\mu$m diameter pore filters. The finished vesicle suspension is then ready for use or may be stored for four or more weeks at 4° to 22°C, frozen or lyophilized. A lipid vesicle suspension containing entrapped chlorambucil-(N-methyl-glucamine) is thus obtained.

## Example 4

Further, DNA is encapsulated into distearoyl:phosphatidylcholine:cholesterol vesicles using calcium chloride.

A dry powder containing a homogenous dispersion of cholesterol in distearoyl phosphatidylcholine at a 1:2 mole ratio is prepared. This dispersion is hydrated with an aqueous solution containing 100 mM calcium chloride at a pH in the range of about 5 to 9. The lipid concentration in the resulting suspension is about 20 to 25 mg/ml. This mixture is then heated to 65°C and homogenized as described above in Example 1. Following homogenization, the mixture undergoes a buffer exchange and unentrapped calcium chloride is replaced with a low ionic strength medium such as 300 mM lactose.

Next, the vesicle preparation is heated again to about 65°C and an amount of DNA is added (as the sodium salt) to yield a lipid to DNA ratio in the range of about 50:1 to 100:1 or more (w/w), but preferably 65:1. If DNA is added in the free acid form it may be necessary to add sufficient base such as NaOH, NaHCO$_3$ or Na$_2$CO$_3$ to maintain iso pH with the internal phase. The drug-vesicle mixture should be maintained at elevated temperature (about 65°C) for about 10 minutes or more. During this phase of the procedure, DNA will pass through the membrane bilayer to form a salt or complex with the entrapped calcium while the chloride (Cl$^-$) dissipates to the vesicles' exterior aqueous space. If a sufficient lipid:drug ratio has been selected (approximately greater than 50:1), greater than about 90% of the added DNA will become vesicle entrapped. Unentrapped, residual, DNA can be removed by ion-exchange, ultrafiltration, gel permeation chromatography or other suitable means. Following drug entrapment, pH may be adjusted as needed to maintain optimal chemical stability or to minimize vesicle aggregation.

The DNA-vesicle suspension can subsequently be sterilized by passage through 0.45 $\mu$m or, preferably, 0.22 $\mu$m diameter pore filters. Alternatively, magnesium or manganese chloride can be used in place of calcium chloride.

## Example 5

This example details the entrapment of doxorubicin into distearoyl:phosphatidylcholine:cholesterol vesicles using a metal-ion citrate complex, and a membrane ionophore to transfer ions across the membrane bilayer.

A dry powder containing a homogeneous dispersion of cholesterol in DSPC at a 1:2 molar ratio was prepared. This dispersion was hydrated with an aqueous 50 mM solution of a metal ion complexed with citrate (i.e. K$^+$, Mg$^{+2}$, Cu$^{+2}$, Al$^{+3}$, Fe$^{+3}$) at a pH of about 6.5. The lipid concentration in the resulting suspension was about 25 mg/ml. This mixture was heated to 65°C and homogenized as described above and cooled to room temperature.

Next, unentrapped metal-citrate complex was removed by buffer exchange using suitable means such as described in Example I (above) and substituting 300 mM sucrose. The vesicle preparation was heated again to about 65°C and an amount of doxorubicin was added to yield a lipid to drug ratio of 25:1. If doxorubicin was added in its hydrochloride salt form, it may be necessary to add a sufficient amount of base such as NaHCO$_3$, or Na$_2$CO$_3$ to maintain a constant pH, approximately 6.5. Alternatively, it may be possible to add the drug in its carbonate salt form. The drug-vesicle mixture should be maintained at 65°C for about 10 minutes. In order to facilitate the transportation of the metal ions across the vesicle membrane, a suitable ion shuttle may be added in small amounts (5-10 $\mu$g/ml) to the sample. Examples of ion shuttles include but are not limited to acetylacetone, hexafluoroacetylacetone, or a-aminopent-3-en-2-one. These shuttles can be removed from the membrane bilayer at a later time by lyophilization or by heating. In general, any $\beta$-diketone or $\beta$-ketoamine could be used as an ion shuttle. The exchange of the metal ions with the doxorubicin then takes place. The ratio of metal ion to doxorubicin exchanged was variable depending on the charge of the metal ion. To help prevent the metal ions from re-entering the vesicles, small quantities (1 mM) of metal chelating agents such as EDTA or a cation exchange resin may be introduced to the sample. The remainder of the processing steps are similar to those described in Example I. A lipid-vesicle suspension containing liposome entrapped doxorubicin-citrate was thus obtained.

From the foregoing description the essential characteristics of the invention can be readily ascertained and, without departing from the spirit and scope thereof, the invention can be adapted to various usages. Changes in form and the substitution of equivalents are contemplated as circumstances may suggest or render expedient, in the context of the following claims.

## Claims

1. Unilamellar liposomes having a diameter of from 30 to 150 nm comprising a liposomal membrane including distearoylphosphatidylcholine (DSPC) and cholesterol, in a DSPC:cholesterol molar ratio of about 2:1, the liposomes encapsulating an antineoplastic selected from the group consisting of vincristine and vinblastine and an anion of low liposomal membrane permeability including a carboxylate moiety; and the liposomes being obtainable by a method comprising forming the liposomes in an aqueous medium including a metal free cation of high liposomal membrane permeability containing carbon and nitrogen and the anion of low liposomal membrane permeability to produce liposomes which include a portion of the medium containing the cation and anion in the interior aqueous space and which are dispersed in the aqueous medium which is external to the liposomes, lowering the concentration of the cation and anion in the external aqueous medium, and adding the antineoplastic to the dispersion of liposomes.

2. The liposomes of claim 1, in which the anion is a monocarboxylate.

3. The liposomes of claim 1, in which the anion is a dicarboxylate.

4. The liposomes of claim 1, in which the anion is a tricarboxylate.

5. The liposomes of claim 1, in which the anion is a polycarboxylate.

6. The liposomes of claim 1, in which the anion is selected from the group consisting of gluceptate, fumarate, gluconate, lactobionate, maleate, succinate, glutarate, tartrate, citrate and carbopol.

7. The liposomes of claim 6, in which the anion is lactobionate.

8. The liposomes of any of claims 1 to 7, in which the liposomal membrane further includes distearoylphosphatidylglycerol (DSPG).

9. The liposomes of any of claims 1 to 8, in which the diameter is from about 45 to about 60 nm.

10. The liposomes of any of claims 1 to 9, obtainable by a method comprising forming the liposomes in an aqueous medium including a cation selected from protonated forms of methylamine, an ethylamine, a propylamine, ethylenediamine or ethanolamine to produce liposomes which include a portion of the medium containing the cation and anion in the interior aqueous space and which are dispersed in the aqueous medium which is external to the liposomes, lowering the concentration of the cation and anion in the external aqueous medium, and adding the antineoplastic to the dispersion of liposomes.

11. Unilamellar liposomes having a diameter of from 30 to 150 nm comprising a liposomal membrane including distearoylphosphatidylcholine (DSPC) and cholesterol in a DSPC:cholesterol molar ratio of about 2:1, the liposomes encapsulating an anion of low liposomal membrane permeability including a carboxylate moiety, and an antineoplastic selected from the group consisting of vincristine and vinblastine.

12. The liposomes of claim 11, in which the anion is a monocarboxylate.

13. The liposomes of claim 11, in which the anion is a dicarboxylate.

14. The liposomes of claim 11, in which the anion is a tricarboxylate.

15. The liposomes of claim 11, in which the anion is a polycarboxylate.

16. The liposomes of claim 11, in which the anion is selected from the group consisting of gluceptate, fumarate, gluconate, lactobionate, maleate, succinate, glutarate, tartrate, citrate and carbopol.

17. The liposomes of claim 16, in which the anion is lactobionate.

18. The liposomes of any of claims 11 to 17, in which the liposomal membrane further includes distearoylphosphatidylglycerol (DSPG).

19. The liposomes of any of claims 11 to 18, in which the diameter is from about 45 to about 60 nm.

20. The liposomes of any of claims 1 to 19, for use as medicaments.

21. The liposomes of any of claims 1 to 20, for use as vehicles to target the delivery of the antineoplastic *in vivo* specifically to tumor cells.

22. Use, in the preparation of pharmaceutical compositions for targeting the delivery of an antineoplastic selected from vincristine and vinblastine *in vivo* specifically to tumor cells, of unilamellar liposomes having a diameter of from 30 to 150 nm comprising a liposomal membrane including distearoylphosphatidylcholine (DSPC) and cholesterol in a DSPC:cholesterol molar ratio of about 2:1 and encapsulating the antineoplastic and an anion of low liposomal membrane permeability including a carboxylate moiety.

23. Use, for encapsulating an antineoplastic selected from vincristine and vinblastine to be targeted specifically to tumor cells *in vivo*, of unilamellar liposomes having a diameter of from 30 to 150 nm comprising a liposomal membrane including DSPC and cholesterol in a DSPC:cholesterol molar ratio of about 2:1 and encapsulating also an anion of low liposomal membrane permeability including a carboxylate moiety.

24. The use of claim 22 or 23, in which the anion is a monocarboxylate.

25. The use of claim 22 or 23, in which the anion is a dicarboxylate.

26. The use of claim 22 or 23, in which the anion is a tricarboxylate.

27. The use of claim 22 or 23, in which the anion is a polycarboxylate.

28. The use of claim 22 or 23, in which the anion is selected from the group consisting of gluceptate, fumarate, gluconate, lactobionate, maleate, succinate, glutarate, tartrate, citrate and carbopol.

29. The use of claim 28, in which the anion is lactobionate.

30. The use of any of claims 22 to 29, in which the liposomal membrane further includes distearoylphosphatidylglycerol (DSPG).

31. The use of any of claims 22 to 30, in which the diameter is from about 45 to about 60 nm.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | WO-A-88 06442 (THE LIPOSOME COMPANY) * page 52 - page 53; example 27 * | 1-31 | A61K9/127 A61K31/475 |
| Y | WO-A-90 03781 (LEGROS ET AL.) * the whole document * | 1-31 | |
| A | US-A-4 923 876 (FRANCIS ET AL.) * the whole document * | 1-31 | |

TECHNICAL FIELDS SEARCHED (Int.Cl.5)

A61K

The present search report has been drawn up for all claims

| Place of search | Date of completion of the search | Examiner |
|---|---|---|
| THE HAGUE | 11 April 1995 | Benz, K |